(19) Europäisches Patentamt
European Patent Office
Office européen des brevets

(11) **EP 1 363 020 A2**

(12) **EUROPÄISCHE PATENTANMELDUNG**

(43) Veröffentlichungstag:
**19.11.2003 Patentblatt 2003/47**

(51) Int Cl.⁷: **F04B 19/00**

(21) Anmeldenummer: 03010741.1

(22) Anmeldetag: **14.05.2003**

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **16.05.2002 DE 10222228**

(71) Anmelder:
• **Roche Diagnostics GmbH**
  **68305 Mannheim (DE)**
  Benannte Vertragsstaaten:
  **DE**
• **F. HOFFMANN-LA ROCHE AG**
  **4070 Basel (CH)**
  Benannte Vertragsstaaten:
  **AT BE BG CH CY CZ DK EE ES FI FR GB GR HU IE IT LU MC NL PT RO SE SI SK TR**

(72) Erfinder:
• **Effenhauser, Carlo, Dr.**
  **69469 Weinheim (DE)**
• **Harttig, Herbert, Dr.**
  **67122 Altrip (DE)**
• **Ziegler, Tony**
  **76889 Steinfeld (DE)**

(54) **Mikropumpe mit Heizelementen für einen pulsierten Betrieb**

(57) Die Erfindung offenbart eine Mikropumpe mit Heizelementen, wobei ein im Wesentlichen pulsierter Betrieb der Heizelemente mittels einer Steuereinheit erfolgt. Aufgrund des pulsierten Betriebes der Heizelemente ist eine exakte Steuerung der Flussrate möglich. Mit Hilfe der Steuereinheit kann die Flussrate über längere Tage hinweg konstant gehalten oder eine Veränderung der Flussrate schnell und präzise durchgeführt werden.

Fig. 1

EP 1 363 020 A2

**Beschreibung**

Die vorliegende Erfindung betrifft eine Mikropumpe sowie die Steuerung einer Mikropumpe für niedrige Flussraten, insbesondere im Bereich von 1 bis 1000 nl/min. Die erfindungsgemäße Pumpe führt ein diskontinuierliches Betreiben eines Heizelementes durch und erzeugt trotz einer gepulsten Betriebsart konstante Flussraten. Mit Hilfe der Mikropumpe kann ein Flüssigkeitstransport über einen Zeitraum von mehreren Tagen mit einer konstanten Flussrate aufrecht erhalten werden, wobei die Flussrate bei Bedarf schnell verändert und präzise neu eingestellt werden kann.

Zur Erfindung gehören weiterhin ein Mikrodialyse- und ein Ultrafiltrationssystem mit einer wie vorstehend genannten Pumpe sowie Verfahren zum Betreiben der Pumpe.

Als mögliche Anwendungen der erfindungsgemäßen Mikropumpe wird im Folgenden beispielhaft die Mikrodialyse und die Ultrafiltration genannt, wobei sich eine Anwendung der Mikropumpe generell in dem Bereich der medizinischen Diagnostik von besonderem Vorteil erweist.

Das Prinzip der Mikrodialyse wird im Folgendem anhand der Erkrankung Diabetes veranschaulicht, bei der es einen vielversprechenden Ansatz zur kontinuierlichen Glucoseüberwachung liefert.

Gegenwärtig leiden über 140 Millionen Menschen weltweit an Diabetes. Schätzungen zufolge wächst die Zahl an erkrankten Menschen in den nächsten 25 Jahren auf ca. 300 Millionen. Unzureichende Kontrollmaßnahmen des Blutglucosewertes der an Diabetes erkrankten Menschen können über die Jahre hinweg zu Langzeitkomplikationen führen. Dabei können z. B. Netzhauterkrankungen, die zur Erblindung führen, sowie Nieren-, Nervenkrankheiten oder Erkrankungen der Herzkranzgefäße entstehen. Ein anderes Risiko ist ein sogenannter hypoglykämischer Schockzustand bei zu niedrigen Blutzuckerwerten, der insbesondere nachts tödlich verlaufen kann. Gegenwärtig werden Blutzuckerkontrollen mit Stechhilfen und Teststreifen durchgeführt. Nachteile sind hier vor allem, dass starke Schwankungen des Blutzuckerspiegels (z. B. Nahrungsaufnahme) wegen der zu seltenen Glucosewerterfassung (ca. 1 - 4 Messwerte pro Tag) übersehen werden können. Aus den erwähnten Gründen strebt man bei Risikopatienten (z. B. bei insulinpflichtigen Diabetikern) eine kontinuierliche Überwachung des Blutzuckerspiegels an. Von großer Bedeutung ist dabei, dass gefährliche Zustände schnell erkannt werden und Gegenmaßnahmen ergriffen werden können.

Einen sehr vielversprechenden Ansatz zur kontinuierlichen Glucoseüberwachung basiert auf dem Prinzip der Mikrodialyse. Dabei diffundiert Glucose aus der interstitiellen Flüssigkeit durch eine Dialysemembran in eine Dialyselösung. Ein Katheter beinhaltet die Membran und ist in das subcutane Fettgewebe des Patienten eingeführt. Stromabwärts des Katheters befindet sich ein elektrochemischer Glucosesensor. Eine ausreichend lange Verweilzeit der Dialyselösung innerhalb des Austauschbereichs der Membran vorausgesetzt, passt sich die Glucosekonzentration in der Dialyselösung sehr gut an die Glucosekonzentration in der interstitielle Flüssigkeit an, so dass die Glucosekonzentration genau bestimmt werden kann. Ein derartiger Katheter wird üblicherweise für einige Tage benutzt, sodass wichtige Forderungen an eine Mikropumpe in dem beispielhaft genannten Anwendungsgebiet gestellt werden. Einige Anforderungen sind z. B. eine konstante Flussrate über einen Zeitraum von mehreren Tagen, eine präzise und direkte Steuerung der Mikropumpe, ein geringer Energieverbrauch, geringe Herstellungskosten bei der Verwendung der Mikropumpe als Einwegprodukt sowie Sterilisierbarkeit, um nur einige Anforderungen zu nennen.

Im Stand der Technik sind miniaturisierte Pumpen bekannt, z. B. Schlauchquetschpumpen, mit denen abhängig von den verwendeten Schlauchdurchmessern Flussraten bis hinab zu etwa 100 nl/min erzielt werden können. Im Focus der Entwicklung von miniaturisierten Pumpen steht im Stand der Technik meistens eine möglichst hohe Förderrate bei minimalem Pumpenvolumen. Es hat sich jedoch gezeigt, dass derartige Pumpen bei Langzeitanwendungen im unterem Förderungsbereich nicht genügend zuverlässig arbeiten, größere Schwankungen der erzielten Flussraten kaum zu vermeiden sind und insbesondere eine kostengünstige Fertigung als Einwegprodukt eine enorme Hürde darstellt.

Sowohl im Bereich der Mikrodialyse als auch der Ultrafiltration sind weiterhin Anordnungen bekannt, bei denen ein Unterdruck-Reservoir (beispielsweise eine aufgezogene Spritze) über eine Kapillardrosselstrekke mit einem Fluidsystem verbunden ist. Nachteilig ist jedoch hierbei der nicht lineare Druckverlauf über die Zeit, der nur über einen ebenso nachteiligen großen Volumenbedarf des Unterdruck-Reservoirs abgemildert werden kann.

Eine weitere Anordnung zur Erzielung kleiner Flussraten ist aus dem Dokument EP-B 0 722 288 bekannt. Bei dieser Anordnung wird eine in einem Kanal befindliche Flüssigkeit mit einem Sorptionsmittel direkt in Kontakt gebracht. Ein solches System weist typischerweise Flussraten im Bereich von wenigen Mikrolitern pro Minute auf.

Das Dokument EP 0 568 024 offenbart eine Pumpe, bei der Heizelemente außerhalb einer Kapillare positioniert sind. Bei Aktivierung der Heizelemente wird Flüssigkeit im Kapillarende verdampft, so dass ein Pumpeffekt resultiert.

Nachteil dieser Pumpe ist jedoch, dass sich das Kapillarende durch den Heizvorgang erhitzt und nur durch einen aufwendigen Temperaturregelmechanismus eine konstante Flussrate gewährleistet werden kann. Bei einer Veränderung der Flussrate ist ebenfalls ein Temperaturregelmechanismus notwendig, der die Flussrate durch Veränderung der Temperatur der Heizelemente variiert. Weiterhin zeigt sich, dass der Zusammenhang zwischen Temperatur und Flussrate nichtlinear und materialabhängig ist. Die Wärmeleitfähigkeit sowie die Wärmekapazität von Flüssigkeit und Kapillare sind dabei wesentliche Faktoren, die die Temperaturabhängigkeit der Flussrate beeinflussen und eine präzise Regelung erschweren. Derartige Temperaturregelmechanismen erlauben daher nur eine langsame Veränderung der Flussgeschwindigkeit, da diese der Trägheit des Regelmechanismusses unterliegt.

Obwohl bereits mehrere Arten von Mikropumpen existieren, erfüllen die im Stand der Technik bekannten Pumpen oftmals nur mangelhaft die genannten Anforderungen. Insbesondere erweist sich eine einfach handhabbare, kostengünstige und exakte Steuerung der Mikropumpen oftmals als problematisch.

Aufgabe der vorliegenden Erfindung ist es, eine Mikropumpe sowie ein Verfahren zum Betreiben der Mikropumpe bereitzustellen, die den genannten Anforderungen genügen, wobei insbesondere eine einfach zu handhabende Steuerung der Flussrate ermöglicht werden soll.

Die erfindungsgemäße Mikropumpe mit einem Heizelement beinhaltet ein fluidisches Transportelement, welches mit einem Flüssigkeitsreservoir verbunden werden kann und im angeschlossenen Zustand das Fluidreservoir mit einem Verdampfungsbereich fluidisch verbindet. Das Heizelement ist im Bereich eines Endes des fluidischen Transportelementes positioniert. Die Pumpe beinhaltet weiterhin eine Steuereinheit zur Steuerung des Heizelementes. Aufgrund der von der Steuereinheit ausgesendeten Signale an das Heizelement befindet sich das Heizelement abwechselnd für einen Zeitraum T1 in einer Ruhephase und für einen Zeitraum T2 in einer Heizphase. Ruhe- und Heizphase wechseln sich im wesentlichen periodisch ab, sodass ein pulsierter Betrieb des Heizelementes erfolgt.

Weiterhin ist eine Mikropumpe mit den genannten Eigenschaften Gegenstand der Erfindung, bei der das fluidische Transportelement mit einem Flüssigkeitsreservoir verbunden ist, und das Transportelement mit Flüssigkeit gefüllt ist.

Im Auslieferungszustand weist die erfindungsgemäße Pumpe vorzugsweise keinen direkten Kontakt der Flüssigkeit mit dem Transportelementende auf, um einen unnötigen Verbrauch von Flüssigkeit durch eine Verdampfung der Flüssigkeit bei Umgebungstemperatur zu vermeiden. Der Kontakt der Flüssigkeit mit dem Transportelementende kann vom Benutzer beispielsweise durch einen gezielten Druckstoß bei Inbetriebnahme der Pumpe erzeugt werden. Durch gezieltes Heizen des Transportelementendes kann nun dosiert Flüssigkeit verdampft werden.

Die erfindungsgemäße Mikropumpe zeichnet sich durch konstante, schnell einstell- und veränderbare Flussraten mit hoher Reproduzierbarkeit aus.

Weiterhin zeichnet sich die Mikropumpe dadurch aus, dass sie einfach und kostengünstig herzustellen ist und Vorzugsweise mit der Herstellung mikrofluidischer Systeme basierend auf Planartechnologie (z. B. Mikrotechnik) kompatibel ist.

Die Mikropumpe zeichnet sich darüber hinaus durch die Kompatibilität mit mehreren Sterilisationsverfahren, wie z. B. β-, γ-, Dampf- und ETD-Gas-Sterilisation, aus.

Durch den Verdampfungsprozess wird Flüssigkeit aus dem Transportelement entfernt, sodass eine gewünschte Pumpwirkung durch nachströmende Flüssigkeit hervorgerufen wird.

Unter dem Begriff Verdampfung ist im Sinne der Erfindung sowohl eine Verdunstung der Flüssigkeit unterhalb ihres Siedepunktes, als auch eine Verdampfung der Flüssigkeit oberhalb ihres Siedepunktes gemeint.

Die Pumpe kann beispielsweise zum Transport einer Flüssigkeit verwendet werden, die als Perfusionsflüssigkeiten im Rahmen einer Mikrodialyse eingesetzt wird. Hierbei strömt die Perfusionsflüssigkeit durch das Mikrodialysesystem, wobei ein zu bestimmender Analyt in die Perfusionsflüssigkeit aufgenommen wird und stromabwärts von der Mikrodialysemembran vermessen wird. Bei Anwendung der Pumpe im Gebiet der Ultrafiltration wird durch die Verdampfung einer Flüssigkeit ein Unterdruck im fluidischen Transportelement erzeugt, der ein weiteres Fluid aus der Umgebung in das fluidische Transportelement hineinbefördert. Bei der Ultrafiltration handelt es sich bei diesem weiteren Fluid um interstitielle Flüssigkeit, die durch eine Ultrafiltrationsmembran aus dem Körper des Patienten in den Kanal eintritt. Beispielsweise wird die zu verdampfende Flüssigkeit an die interstitielle Flüssigkeit fluidisch angekoppelt, so dass ein Transport der interstitielle Flüssigkeit bewirkt wird. Die Fluide liegen dann im Fluidkanal segmentiert von einander vor. Die fluidische Ankopplung kann beispielsweise durch eine Luftblase oder eine mit beiden Flüssigkeiten nicht mischbare Flüssigkeit, wie z. B. in EP 1 167 757 beschrieben, ermöglicht werden.

Im Rahmen der Erfindung können beliebige Flüssigkeiten eingesetzt werden, bevorzugt werden jedoch wässrige Flüssigkeiten verwendet. Neben dem Wasseranteil können die wässrigen Flüssigkeiten, Stoffe oder Stoffgemische enthalten, die die Oberflächenspannung und/oder Viskosität beeinflussen, sodass die Flussrate der Flüssigkeit im fluidische Transportelement beeinflusst wird. Vorzugsweise enthalten die Flüssigkeiten in einem Temperaturbereich, in dem die Pumpe arbeitet, keine oder nur geringe Mengen unverdampfbare Substanzen, wie z. B. Salze, da sich diese durch den Verdampfungsprozess am Transportelementende anreichern und zu einer Verstopfung des fluidischen Transportelementes führen können. Vorteilhafterweise werden daher als Flüssigkeiten nur vollständig verdampfbare Komponenten, z. B. Alkohole, verwendet.

Bei der erfindungsgemäßen Pumpe befindet sich das Heizelement im Bereich des fluidischen Transportelementendes, das an diesem Ende so beschaffen ist, dass verdampfte Flüssigkeit aus dem Transportelemente austreten kann. Im Rahmen der Erfindung ist der Bereich des fluidischen Transportelementendes dadurch begrenzt, dass das Heizelement maximal nur soweit in das Innere des fluidischen Transportelementes hineinragt, das beim Heizen und Verdampfen der Flüssigkeit im Wesentlichen keine Blasenbildung im Transportelement erfolgt, die zum Herausschleudern von unverdampfter Flüssigkeit aus dem Transportelement führen kann. Es wird so gewährleistet, dass nur verdampfte Flüssigkeit aus dem Transportelement gelangt.

Die erfindungsgemäße Mikropumpe zeichnet sich dadurch aus, dass das Prinzip des gepulsten Betriebes eines Heizelementes eine dosierte Verdampfung der Flüssigkeit bewirkt, aus der ein für die Anwendung genügend kontinuierlicher Pumpeffekt resultiert, sodass eine einfache und exakte Steuerung der Flussrate ermöglicht wird. Das Prinzip wird nachfolgend kurz verdeutlicht.

Zu Beginn befindet sich das Heizelement in einer Ruhephase, T1. Hierbei ist das fluidische Transportelement im Wesentlichen vollständig mit der zu transportierenden Flüssigkeit gefüllt. Für einen Zeitraum, T2, wird das Heizelement aktiviert, sodass sich in der Heizphase das Ende des Transportelementes erhitzt. Durch die freigesetzte Wärme wird Flüssigkeit im Bereich des Transportelementendes verdampft. Die Menge der während T2 verdampften Flüssigkeit ist vom Befüllungsgrad des Kapillarendes am Heizelement, von der Dauer der Heizphase T2, von der zugeführten Wärmemenge sowie vom Dampfpartialdruck der Flüssigkeit in der Atmosphäre abhängig. Während der Heizphase T2 werden einzelne, diskrete Flüssigkeitsportionen durch Verdampfen in die Gasphase überführt. Durch Veränderung der Parameter T1, T2 und / oder der Heizleistung lässt sich die mittlere freigesetzte Wärme exakt dosieren, so dass eine präzise Steuerung der jeweils verdampfenden Flüssigkeitsmenge möglich ist.
Die im Wesentlichen vollständig verdampfte Flüssigkeit entweicht aus dem Transportelement. Aufgrund von z. B. Kapillarkräften im Inneren des Transportelementes wird Flüssigkeit aus dem Flüssigkeitsreservoir ins Transportelementende gezogen. Ist die Heizphase T2 abgeschlossen, kann sich in der Ruhephase T1 das Transportelement wieder vollständig füllen. Es ist jedoch auch denkbar, dass sich ein nicht vollständiges Füllen des Transportelementes als sinnvoll erweist; hierauf wird im Folgenden näher eingegangen. Anschließend wird erneut das Heizelement aktiviert, sodass wiederum eine definierte Flüssigkeitsportion verdampft wird. Werden die Heizimpulse sehr kurz hintereinander an das Heizelement ausgesendet ( kleine T1-Zeiten), besteht nicht hinreichend Zeit, um ein vollständiges Auffüllen des Transportelementes wieder zu gewährleisten. Ein erneuter Heizimpuls kann folglich nur maximal die Flüssigkeitsmenge verdampfen, die in der Ruhephase in das Transportelementende nachgeflossen ist. Eine Veränderung der Ruhephase, T1, und somit der Zeitspanne zwischen zwei aufeinanderfolgenden Verdampfungsvorgängen kann folglich die Menge der verdampften Flüssigkeit beeinflussen. Hierbei erfolgt der Einfluss durch die Regelung des Befüllungsgrades des Kapillarendes bei Unterschreitung der Mindestzeit zur vollständigen Befüllung.

Durch Steuerung der Heizphase und der Ruhephasen ist somit eine direkte Beeinflussung des Verdampfungsprozesses möglich. Die Steuerung des Verdampfungsprozesses erfolgt hierbei in der Weise, dass ein für die Anwendung genügend kontinuierlicher Flüssigkeitstransport aus dem Flüssigkeitsreservoir in das Transportelement resultiert, sodass sich eine gewünschte Flussrate einstellt. Eine gewünschte Flussrate wird demzufolge durch die Länge der Heizphase T2, der Ruhephase T1 sowie der Heizleistung gesteuert.

Im Rahmen der Erfindung zeichnet sich folglich eine gepulste Betriebsart dadurch aus, dass diese durch die genannten Parameter charakterisierbar ist und eine Kombination der genannten Parameter einer jeweiligen verdampften Flüssigkeitsmenge und somit einer Flussrate entspricht. Im Gegensatz zum Stand der Technik, in dem sich das Heizelement aufgrund eines Temperaturregelmechanismus ebenfalls in einer Ruhe- oder Heizphase befinden kann, wird jedoch Ruhe- und Heizphase nicht wie im Stand der Technik durch einen a-periodischen Temperaturregelmechanismus bestimmt, sondern dient zur direkten Steuerung der Flussrate.

Vorteilhaft erfolgt die gepulste Betriebsart in der Weise, dass das Heizelement in weniger als 20 % der Betriebszeit der Pumpe aktiviert ist.

In einer vorteilhaften Ausführungsform ist mindestens einer der Parameter (T1, T2, Heizleistung) durch die Steuereinheit der Mikropumpe veränderbar, sodass die Flussrate mittels der Steuereinheit einstellbar ist. Die Steuereinheit kann beispielsweise wiederum über ein Programm selbst gesteuert werden, das z. B. einen cyclischen Flussratenverlauf vorgibt, der einem entsprechenden Anwendungsgebiet angepasst ist. Hierbei ist es z. B. denkbar, dass die Flussrate zum Aufnehmen des Analyten in das Fluid cyclisch in einem vorbestimmten Zeitintervall verlangsamt und wieder beschleunigt wird. Es ist jedoch auch denkbar, dass der Benutzer der Mikropumpe selbst die Steuereinheit programmiert bzw. die entsprechenden Parameter bei Bedarf verändert.

Im Vergleich zu einem kontinuierlichen Betrieb eines Heizelementes ist durch die beschriebenen Beziehungen zwischen T1, T2 und der Heizleistung eine exakte Steuerung der Flussrate möglich. Ein Herabsetzen der Flussrate beispielsweise muss nicht durch eine Temperaturerniedrigung von Heizelementen erfolgen, die häufig einen aufwendigen Regelkreislauf zum Einstellen einer vorgegebenen Temperatur benötigen, sondern erfolgt durch Modulation einen oder mehrere genannten Parameter (T1, T2, Heizleistung). Eine Flussratenveränderung ist folglich direkt und ohne durch regelmechanismusbedingten Verzögerungen steuerbar. Des weiteren verhindert der pulsierte Betrieb des Heizelementes ein übermäßiges Aufheizen des Transportelementes, da während der Ruhephasen, T1, eine Abkühlung erfolgt. In einer vorteilhaften Ausführungsform wird die Ruhephase, T1, so gewählt, dass das Transportelement im Wesentlichen auf seine Umgebungstemperatur abkühlt. Dies unterstützt eine präzise Steuerung der Flussrate, da ein Aufheizen des Transportelementendes bei kontinuierlichen Betrieb beispielsweise zu einer höheren unkontrollierten Grundverdampfungsrate führt.

In einer vorteilhaften Ausführungsform wird, um ein übermäßiges Aufheizen des Transportelementes zu vermeiden, zusätzlich die Heizphase T2 in Signalpulse unterteilt. Dies bewirkt neben einer Präzisierung der erzeugten Wärmemenge weiterhin ein energiesparsames Betreiben der Mikropumpe, das bei batteriebetriebenen Pumpen von besonderem Vorteil ist. Der Abstand der Pulse innerhalb der Heizphase wird hierbei so kurz gewählt, dass nahezu kein Befüllen des Transportelementendes durch nachfließende Flüssigkeit erfolgt, ohne dass eine sofortige Verdampfung dieser resultiert. Im Rahmen der Erfindung ist die Heizphase im Vergleich zur Ruhephase dadurch gekennzeichnet. Erst während der Ruhephase findet ein Nachfließen der Flüssigkeit in dem Maße statt, dass das Transportelementende erneut mit Flüssigkeit gefüllt wird.

Die Heizphase kann folglich durch ein einziges Signal gesteuert werden oder, wie beschrieben, durch mehrere hintereinander folgende, gepulste Signale. Für die erfindungsgemäße Mikropumpe ist es dabei zunächst nicht entscheidend auf welche Art das Heizen initiiert wird. Es zeigt sich, dass eine Steuerung der Wärmemenge neben den bereits genannten Möglichkeiten durch eine Variation von Pulslängen sowie von Abständen zwischen Pulsen innerhalb der Heizphase eine verbesserte Steuerung der Flussrate leistet.

Zur Steuerung der Heiz- und Ruhephasen sind prinzipiell alle möglichen Kombinationen von im Wesentlichen periodisch abwechselnden Pulsen denkbar, mit deren Hilfe eine konstante Flussrate eingestellt werden kann.

Ein pulsierter Betrieb einer Mikropumpe kann sowohl bei einer niedrigen Verdunstungsrate des Mikropumpensystems bei Umgebungstemperatur erfolgen, sodass eine exakte Steuerung auch niedriger Flussraten erleichtert wird. Es ist jedoch auch ein Betreiben bei einer höheren Verdunstungsrate denkbar, bei der die Pumpe aufgrund ihrer Verdunstungsrate in der Ruhephase eine erhöhte Flussrate besitzt, die durch die Verdunstung der Flüssigkeit bei Umgebungstemperatur der Pumpe verursacht wird. Die Verdunstungsrate der Flüssigkeit einer Pumpe, die im Wesentlichen durch die Umgebungstemperatur verursacht wird, wird im Rahmen der Erfindung als Basis- oder Grundflussrate bezeichnet. Diese kann dann durch einen pulsierten Betrieb des Heizelementes wiederum erhöht werden. Ein Beispiel für eine Mikropumpe mit einer hohen Verdunstungsrate ist z. B. in der Patentanmeldung EP 1 167 757 beschrieben. Eine solche Pumpe besitzt den Vorteil, dass sie besonders energiearm betrieben werden kann, da die Bereitstellung der Basisflussrate bei genügender Temperaturkonstanz der Umgebung keine zusätzliche Energiequelle benötigt.

Grundsätzlich zeigt sich, dass für die Anwendung der erfindungsgemäß pulsierten Betriebsart einer Mikropumpe vielfältige Materialwahl, Materialkombination sowie Pumpendesign denkbar sind, sodass die Anwendung auf kein spezifisches Pumpenmodel mit einem Heizelement beschränkt ist. Hierbei wird der Energieverbrauch der Mikropumpe durch eine pulsierte Betriebsart im Vergleich zu einem kontinuierlichen Betrieb weiterhin verringert.

Die Erfindung ermöglicht ein neues Prinzip einer verdampfungsbasierten Mikropumpe für niedrige Flussraten. Die erfindungsgemäße Mikropumpe besitzt trotz ihrer Einfachheit eine hohe Zuverlässigkeit und niedrige Herstellungskosten.
Die Abhängigkeit der erfindungsgemäßen Mikropumpe von äußeren Einflüssen, wie z. B. der Umgebungstemperatur oder der Luftfeuchtigkeit wird im Vergleich zum Stand der Technik verbessert, wobei insbesondere bei bevorzugten Ausführungsformen mit einem Sorptionsmittel in einem abgeschlossenen Gasraum über dem Kanal, aus dem das Fluid verdampft, eine Minimierung der äußeren Einflüsse gegeben ist.

Wie bereits oben erwähnt, ist bei einer vorteilhaften Ausführungsform einer Mikropumpe die Signallänge, Heizleistung und/oder der zeitliche Abstand zwischen zwei Signalen durch die Steuereinheit veränderbar. Dabei ist sowohl die Veränderung einer sowie mehrerer Größen denkbar. Durch eine diskontinuierliche Steuerung des Heizelementes, bei der z. B. Pulse während der Heizphase durch die Steuereinheit variiert werden sowie deren Abstände, ist eine Unterteilung des verdampften Fluids in Inkremente zulässig, sodass in einer bevorzugten Ausführungsform eine digitalisierte Steuerung der Flussrate mittels der Steuereinheit möglich wird. Beispielsweise erfolgt dann die Steuerung durch elektrische Signale der Steuereinheit. Bei einer bevorzugten Ausführungsform werden die Signale der Steuereinheit an eine Widerstandsheizung als Heizelement weitergeleitet. Es ist jedoch auch jegliche andere Form eines Heizelementes denkbar. So kann z. B. eine geeignete Vorrichtung, die die Flüssigkeit mit Hilfe von elektromagnetischer Strahlung erwärmt, als Heizelement dienen.

Unter fluidischen Transportelementen im Sinne der Erfindung sind alle Strukturen zu verstehen, die einen Transport von Flüssigkeit ermöglichen. Dies kann in einer bevorzugten Ausführungsform ein Kanal sein, der beispielsweise so ausgebildet ist, dass ein Nachfließen zumindest eines Teils des Fluids ins Kanalende durch Kapillarkräfte verursacht wird. Es sind auch mehrere Kanäle oder andere Transportelemente innerhalb einer Mikropumpe denkbar. Beispielsweise können derartige Transportelemente aus verdrillten oder parallelen Einzelfäden, die Litzen bilden, gebildet werden. Eine solche Ausgestaltung des Transportelementes erweist sich für höhere Flussraten als besonders vorteilhaft, da hier in besonderem Maße eine effektive Verdampfung der Flüssigkeit erfolgen kann. Es sind jedoch auch eine Membran oder mikroporöse Strukturen, durch die eine Verdampfung der Flüssigkeit ermöglicht wird, als Transportelemente denkbar. Die genannten Transportelemente können hierbei jeweils über ein Heizelement oder mehrere Heizelemente verfügen. Hierbei kann auch ein Heizelement für mehrere Transportelemente dienlich sein. Bei einer derartigen Ausführungsform kann durch die im Transportelement befindliche Flüssigkeit die erzeugte Wärmemenge weitergeleitet werden, sodass sich die Verwendung eines Heizelementes für mehrere Transportelemente als hinreichend erweist.

Das oder die Heizelemente sind in der Weise innerhalb des Bereiches des Transportelementendes positioniert, dass sich das oder die Heizelemente z. B. innerhalb eines Kanalendes befinden. Es ist jedoch auch möglich, dass die Heizelemente außerhalb des Transportelementendes, aber in direkter Nachbarschaft zu ihm positioniert sind. Beispielsweise sei hier eine Ausführungsform genannt, bei der das Heizelement an ein Kanalende angrenzt, sich jedoch außerhalb des Kanals befindet. Wird das Transportelement aus einer Membran gebildet, kann diese beispielsweise aus einem mit porösem Material gefüllten Kanal bestehen, es ist jedoch auch eine Ausführungsform mit einer Membran denkbar, wie sie beispielhaft in der Patentanmeldung EP 1 167 757 beschrieben ist. Das Transportelement kann vorzugsweise in sequentieller Weise mit einem zweiten weiteren Transportelement fluidisch verbunden sein. Vorzugsweise ist das zweite Transportelement wiederum mit einem zweiten Flüssigkeitsreservoir verbunden, sodass innerhalb des ersten Transportelementes eine Mischung der Flüssigkeiten aus dem ersten und dem zweiten Flüssigkeitsreservoir erfolgt. Diese Ausführungsform ist besonders dann vorteilhaft, wenn die zu transportierende Flüssigkeit schlecht zu verdampfende Substanzen enthält, sodass es zur Aufkonzentration und Ablagerung dieser Substanzen am Transportelementende kommen kann. Eine Verdünnung der zu transportierenden Flüssigkeit mittels eines zweiten Flüssigkeitsreservoirs verhindert somit die Verstopfung des Transportelementes und gewährleistet einen konstanten Flüssigkeitstransport über lange Zeiträume

Das im Wesentlichen vollständig verdampfte Fluid kann beispielsweise aus dem Transportelement in die Umgebung der Mikropumpe entweichen. Vorteilhafterweise besteht jedoch auch die Möglichkeit die verdampfte Flüssigkeit in einen Sorptionsbereich zu leiten, sodass die Umgebung oder wahlweise der Sorptionsbereich mindestens einen Teil des Verdampfungsbereiches der Pumpe darstellt.

Der Sorptionsbereich ist dann außerhalb des fluidischen Transportelementes angeordnet und enthält ein Sorptionsmittel. Das Sorptionsmittel kann hierbei die verdampfte Flüssigkeit aufnehmen, sodass die Flüssigkeit im Wesentlichen vollständig von dem Sorptionsmittel aufgefangen wird und der Dampfdruck im Verdampfungsbereich im Wesentlichen konstant bleibt. Außer vom Dampfdruck des Verdampfungsbereiches der Pumpe hängt die Transportrate der Flüssigkeit. - wie bereits oben erwähnt - von der Temperatur ab, die wiederum unter anderem die Viskosität der Flüssigkeit beeinflusst. Die Viskosität der Flüssigkeit sowie die Oberflächenspannung sinkt mit steigender Temperatur. In entgegengesetzter Richtung wirkt eine steigende Temperatur auf die Verdunstungsrate. Es ergibt sich folglich ein komplexer Zusammenhang zwischen Flussrate und der Temperatur der Umgebung der Mikropumpe, der zu Schwankungen in der Flussrate führen kann. Dies ist besonders bei Pumpen mit einer hohen Basisflussrate der Fall, da die Basisflussrate eine direkte Abhängigkeit zu den Eigenschaften der Umgebung hat. Durch geeignete Wahl des Sorptionsmittels kann jedoch gewährleistet werden, dass die Abhängigkeit von den Umgebungsbedingungen gering ist, sodass durch die beschriebenen bevorzugte Ausführungsform Störeinflüsse im Wesentlichen beseitigt werden.

Ein Beispiel für eine Pumpe mit einem Sorptionsmittel, bei der jedoch die Gesamtmenge der zu transportierenden Flüssigkeit durch die Aufnahmekapazität des Sorptionsmittels begrenzt ist, wird in der Patentanmeldung EP 1 167 757 beschrieben. Das Pumpenkonzept basiert auf dem kontrollierten Transport von Flüssigkeiten durch eine Membran in einen Gasraum, in dem sich ein Sorptionsmittel befindet. Solange es dem Sorptionsmittel in der Kammer gelingt, dem Dampfdruck unter dem Sättigungsdampfdruck zu halten, wird Flüssigkeit aus der Membran in die Kammer verdunstet. Aufgrund der verdunsteten Flüssigkeit wird kontinuierlich durch die Kapillarkräfte in der Membran Flüssigkeit aus einem Flüssigkeitsreservoir nachgeliefert. Eine erfindungsgemäße Ausführungsform einer Mikropumpe in Kombination mit einer analogen Anordnung eines Sorptionsbereiches ermöglicht neben der verbesserten Steuerung der Flussrate die Abhängigkeit vom Dampfdruck zu begünstigen.

Der Begriff "Sorptionsmittel" soll sowohl Adsorbiermittel als auch Absorbiermittel umfassen. Als Sorptionsmittel sind beispielsweise Kieselgele, Molekularsiebe, Aluminiumoxide, Cellulite, Tone, Aktivkohle, Natriumsulfat, Phosphorpentoxid usw. geeignet.

In einer weiteren vorteilhaften Ausführung wird die Umgebungstemperatur der Mikropumpe z. B. in einem Mikrodialyse- oder Ultrafiltrationssystem durch einen engen Kontakt zum menschlichen Körper weitgehend konstant gehalten. Hierbei ist ein direkter Kontakt des Gehäuses, in dem sich die Pumpe befindet, mit der Körperoberfläche von Vorteil, da somit eine annähernd konstante Umgebungstemperatur gewährleistet werden kann. Weiterhin kann eine konstante Temperierung der Umgebung vorteilhafterweise dadurch unterstützt werden, dass die nicht an den Körper anliegende Seite des Pumpensystems thermisch isoliert wird. Eine Abhängigkeit der Flussrate von störenden Einflüssen der Umgebung wird somit ebenfalls weitestgehend minimiert.

Weiterhin ist Gegenstand der Erfindung ein Verfahren zum Betreiben einer Mikropumpe. Durch Aussenden eines ersten Signals wird eine Heizphase initiiert, die eine Wärmeentwicklung innerhalb eines fluidischen Transportelementes einer Mikropumpe im Bereich des Transportelementendes hervorruft. In Abhängigkeit des ausgesendeten Signals findet eine Heizphase für einen definierten Zeitraum T2 statt. Aufgrund der erzeugten Wärmemenge verdampft mindestens ein Teil einer Flüssigkeit, die sich im Inneren des Transportelementes befindet. Die im Wesentlichen vollständig verdampfte Flüssigkeit entweicht aus dem Transportelement. Nach Abschluss der Heizphase kühlt der Bereich des Transportelementendes ab, wobei zumindest ein Teil der Flüssigkeit in das Transportelementende nachfließt. Nach einem bestimmten Zeitraum T1 nach Aussenden des ersten Signals wird ein zweites Signal gesendet, das eine erneute Heizphase initiiert. Erneut wird Flüssigkeit aus dem Transportelement verdampft. Durch wiederholte Initiierung von Heiz- und Ruhephase, wie beschrieben, fließt Flüssigkeit in der Weise in das Transportelementende nach, dass sich eine bestimmte Flussrate einstellt.

Vorteilhafte Ausführungsform des Verfahrens ergeben sich wie bereits beschrieben.

Die Erfindung beinhaltet weiterhin ein Mikrodialyse- sowie ein Ultrafiltrationssystem mit einer Pumpe wie beschrieben, wobei vorteilhafterweise das Mikrodialyse- oder Ultrafiltrationssystem einen stromabwärts der Mikrodialyse- oder Ultrafiltrationsmembran angeordneten Sensor zur Detektion eines oder mehrerer Analyten in der Flüssigkeit besitzt. Bei dem Ultrafiltrationssystem wird - wie bereits beschrieben - durch die Membran Körperflüssigkeit in einen Kanal eingezogen, sodass ein oder mehrere Analyten in der Körperflüssigkeit detektiert werden können. Für eine Mikrodialyse kann direkt Perfusat als zu verdampfende Flüssigkeit eingesetzt werden, das durch einen Mikrodialysekatheter hindurchgeführt wird, um Analyt aufzunehmen. Alternativ ist es möglich, eine Vielzahl von Flüssigkeiten, wie z. B. eine Ringerlösung, vorzusehen, bei denen eine fluidisch Ankopplung erfolgt. Hierbei wird bei der Ultrafiltration der Verdampfungsprozess verwendet, um einen Unterdruck im Kanal zu erzeugen, der Körperflüssigkeit in einen Ultrafiltrationskatheter einzieht.

Die vorliegende Erfindung wird anhand von Figuren näher erläutert.

Figur 1: Mikropumpe mit Heizelement

Figur 2: Mikropumpe mit hoher Grundverdampfungsrate und Sorptionsbereich

Figur 3: Pulsabfolgen während einer Heiz- und Ruhephase

Figur 4: Schaltbild eines Pulsgenerators

Figur 5: Schematische Darstellung eines Pumpenzyklusses

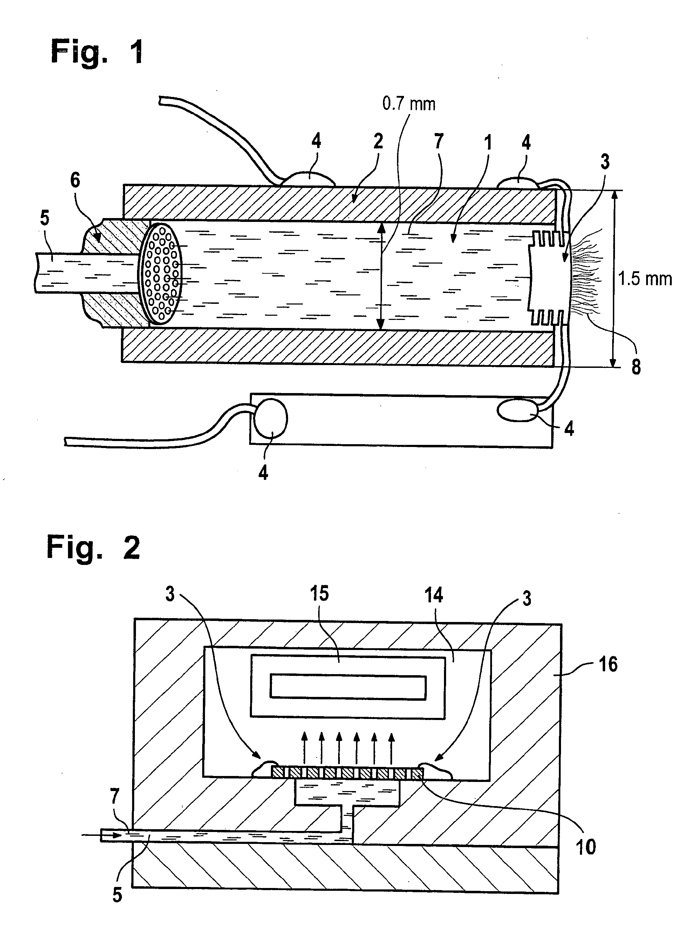

Figur 1 zeigt den Aufbau einer Mikropumpe. Kern der Pumpe ist eine Stahllitze (1), welche sich in einem Messingmantel (2) befindet. Das Heizelement (3) wird von einem gewickelten Konstantandraht, der sich am Ende der Litze (1) befindet, gebildet. Der Widerstand des Konstantandrahtes ist im Wesentlichen temperaturunabhängig, sodass für die Leistung (P) des Heizelementes gilt:

$$P = \frac{U^2}{R} \, ,$$

wobei gilt: U = Spannung und R = Widerstand

Für die gebildete Wärmemenge (Q) gilt dabei in guter Näherung:

$$Q = P \cdot t \quad \text{,wobei t die Zeitdauer ist, für die der Widerstand vom Strom durchflossen wird.}$$

Über die Steuerung der anliegenden Spannung bei ausgewählter Zeitdauer ist somit die gebildete Wärmemenge bestimmbar. Die gebildete Wärmemenge (Q) ist wiederum direkt proportional zum Volumen (V) des verdampften Fluids.

$$V = K \cdot Q$$

Hierbei wird die Proportionalitätskonstante (K) durch die fluidspezifische Wärmemenge bestimmt, die notwendig ist, um das Fluid, ausgehend von der Temperatur des Fluids in der Ruhephase, zu verdampfen.

Die Flussrate (V) ist somit direkt proportioniert zur Heizleistung

$$V = K \cdot P$$

Die Stahllitze (1) bildet das fluidische Transportelement mit einem Querschnitt von ca. 100 μm. Die Heizung (3) ist mit elektrischen Anschlüssen (4) verbunden. Am anderen Ende der Stahllitze ist das fluidische Transportelement mit einem Flüssigkeitsreservoir (5) über einen Kanal verbunden. Die fluidische Verbindung wird mittels eines geeigneten Klebers, wie z. B. eines Epoxydharzes (6) abgedichtet. Im Transportelement (1) befindet sich die zu transportierende Flüssigkeit (7) durch Aktivieren des Heizelementes (3) mittels eines elektrischen Signals findet ein Verdampfen der Flüssigkeit statt, welches in der Figur 1 durch Dampf (8) schematisch dargestellt ist. Hieraus resultiert eine Pumpwirkung, sodass sich eine Flussrate in Abhängigkeit der gebildeten Wärmemenge einstellt. Mit der dargestellten Mikropumpe können z. B. Flussraten zwischen 1 bis 1000 nl/min erzielt werden.

Figur 2 zeigt eine Mikropumpe, bei der das fluidische Transportelement durch eine Membran (10) gebildet wird. Die Membran (10) steht mit einem Fluidreservoir (5) in Verbindung. An der Membran (10) sind zwei Heizelemente (3) positioniert. Beim Aktivieren der Heizelemente wird die gebildete Wärme an den Heizelementen mittels der zu transportierenden Flüssigkeit (7) innerhalb der Membran weitergeleitet. Es findet ein gleichmäßiges Verdampfen der Flüssigkeit aus der Membran in einen Sorptionsbereich (14) statt. Im Sorptionsbereich befindet sich ein Sorptionsmittel (15) das die verdampfte Flüssigkeit absorbiert. Der Sorptionsbereich (14) wird durch das Pumpengehäuse (16) eingeschlossen. Mittels der dargestellten Anordnung einer Mikropumpe ist es möglich, die Funktionsweise der Pumpe im wesentlichen unabhängig von Eigenschaften der Umgebung, wie z. B. der Luftfeuchtigkeit, zu gewährleisten. Aufgrund des Kapillareffektes der Membranstruktur und des Sorptionsmittels, das einen konstanten Dampfdruck im Sorptionsbereich gewährleistet, wird eine Pumpwirkung auch ohne zusätzliches Heizen erzielt, sodass eine Basis- oder Grundflussrate bewirkt wird. Die dargestellte Anordnung ermöglicht somit eine gezielte Steuerung einer Flussrate, die größer ist als die Basis- oder Grundflussrate der Mikropumpe.

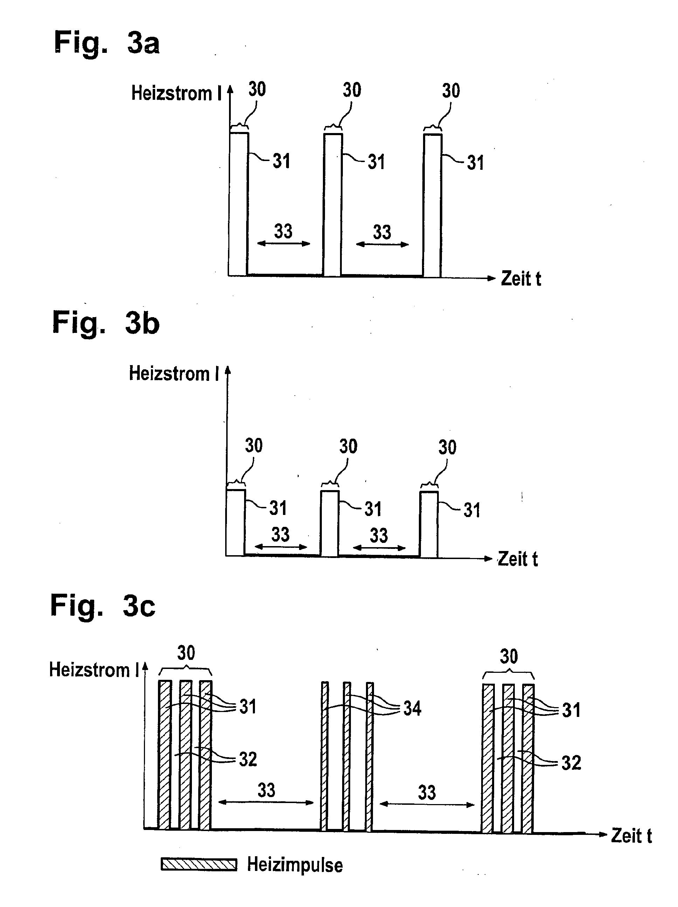

Figur 3a und 3b zeigen beispielhaft eine Pulsfolge mit drei Pulsen (31), die jeweils während der Heizphase T2 (30), durch einen Strom I gebildet werden. Nach der Heizphase (30) wird der Stromfluss unterbrochen, sodass die Heizleistung in der Ruhephase T1 (33) gleich null ist. Die Heizleistung während der Heizphase ist im gezeigten Beispiel konstant. Figur 3a unterscheidet sich zu Figur 3b aufgrund einer reduzierten Heizleistung während der Heizphase. Die Länge der Heiz- und Ruhephasen sind in Figur 3a und 3b identisch. Eine Verringerung der Heizleistung führt unter den dargestellten Bedingungen zu einer Reduzierung der gebildeten Wärmemenge, sodass eine erniedrigte Flussrate im Vergleich zu Figur 3a resultiert.

Figur 3c zeigt beispielhaft eine Pulsabfolge während einer Heiz- und Ruhephase, wobei die Heizphase T2 (30) in drei gleich lange Pulse (31) unterteilt wird bei identischer Leistung. Die Pulse (31) werden durch Pulspausen (32) für eine Zeitdauer, t, unterbrochen. Die Zeitdauer der Pulspause sowie der Pulse ist dabei identisch gewählt. Nach dem letzten Heizpuls beginnt die Ruhephase T1 (33). Die Ruhephase wird unterbrochen durch eine Pulskombination von 3 kurzen Heizpulsen (34), wobei die Pulspausen zwischen den Heizpulsen (34) deutlich länger als die Zeitdauer der Pulse ist. Es folgt erneut eine Ruhephase (33) sowie eine Heizphase (30).

Aufgrund der Heizphase (30) findet eine definierte Wärmeentwicklung im Transportelement statt. Durch die Wahl der kurz hintereinander folgenden Pulse kann die entwikkelte Wärmemenge präzise gesteuert werden, ohne dass ein übermäßiges Aufheizen des Transportelementendes erfolgt. In Folge der Wärmeentwicklung verdampft die im Transportelement vorhandene Flüssigkeit. Hierbei erfolgt während der Heizphase kein erneutes Befüllen des Transportelementendes. Erst während der Ruhephase (33) kommt es erneut zur Befüllung des Transportelementende, wobei Flüssigkeit aus einem Flüssigkeitsreservoir in das Transportelementende nachfließt. Die Heizpulse (34) dienen dazu, eine konstante Pumpenleistung zu gewährleisten, ohne dass es zum Einbruch der Flussrate während der Ruhephasen kommt. Des weiteren vermeiden die Heizpulse (34) Schwankungen der Flussrate, die bei zu lang gewählten Ruhephasen auftreten können. Dies ist durch eine gewisse Trägheit des Systems begründet, da das Nachfließen der verdampften Flüssigkeit erst einige Zeit nach Ende eines Heizimpulses einsetzt. Das Einfügen eines leistungsärmeren Heizblockes, wie es durch die Heizpulse (34) dargestellt ist, ermöglicht folglich eine konstante Flussrate bei minimalem Energieverbrauch. Nach einer wiederholten Ruhephase (33) setzt erneut die Heizphase (30) ein.

Die dargestellten Pulsabfolgen sollen nur ein Beispiel von vielen Möglichkeiten veranschaulichen. Die beschriebene Kombination der Pulse sowie andere Varianten lassen sich dabei leicht in einer entsprechenden Elektronik in der Steuereinheit der Mikropumpe umsetzen.

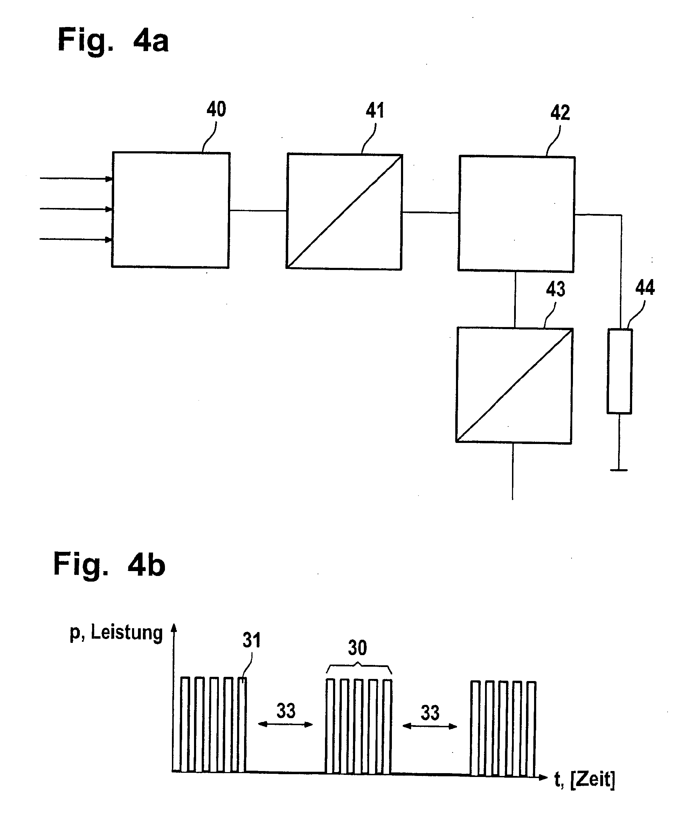

Figur 4 zeigt vereinfacht ein Blockschaltbild, welches beispielhaft zur Steuerung der Heizung verwendet wird. Der Pultsgenerator beinhaltet einen Mikroprozessor (40), der mit einem Digital/Analog-Wandler (41) über eine Leistungsendstufe (42) mit einem Heizelement (44) kontaktiert wird.

Mittels eines Netzteils (43) wird der Pulsgenerator mit Strom gespeist. Zur Erzeugung einer Pulsfolge, wie in Fig. 4 b dargestellt, wird der programmierbare Mikroprozessor in einer für ihn passenden und vom Hersteller bereitgestellten Programmiersprache programmiert. Anhand der schematisch dargestellten Pulsfolge werden nachfolgend die einzelnen Programmierschritte näher erläutert. Hierbei erfolgen die Programmierschritte jeweils mit einer Pause, ohne dass in der Beschreibung dieses jeweils dargestellt wird.

Zuerst wird ein Startbefehl für den Ablauf des Programmes gegeben

- Der Mikroprozessor übermittelt dann einen Spannungswert, der der Leistung während des ersten Pulses entspricht sowie

- die Zeitdauer des ersten Pulses, sodass analog zu Fig. 3 ein Puls (31) definiert wird.

Anschließend wird

- ein Spannungswert, der der Leistung während der ersten Pulspause entspricht sowie

- die Zeitdauer der ersten Pulspause bestimmt und übermittelt.

Zur n-fachen Wiederholung des Pulses wird ein Rekursionsbefehl mittels der genannten Schritte definiert.
Die dargestellten Programmierschritte ergeben somit eine erste Heizphase (30), wie sie bereits in Fig. 3c verbildlicht wurde.
Anschließend wird

- ein Spannungswert, der der Leistung während einer ersten langen Pause entspricht übermittelt sowie

- die Zeitdauer einer ersten langen Pause, sodass es zur Ausbildung einer Ruhephase (33) kommt.

Zur periodischen Wiederholung von Heiz- und Ruhephase wird wiederum ein Rekursionsbefehl durch die dargestellten Schritte charakterisiert.
Soll die Mikropumpe nicht weiter betrieben werden, erfolgt ein Stopbefehl zum Anhalten des Programmablaufs, sodass im Wesentlichen keine Pumpenwirkung oder im Falle einer Mikropumpe mit hoher Verdampfungsrate (s. Fig. 2) die entsprechende Basisflussrate wieder erzielt wird.
Die gemäß der obigen Anweisung vom Mikroprozessor generierte digitale Datenreihe wird vom D/A-Wandler in eine analoge Zeit/Spannungskurve umgesetzt. Diese Zeit/Spannungskurve entspricht der Pulsfolge, wie in Fig. 4b dargestellt.
In der Leistungsendstufe, die ihre Leistung vom Netzteil bezieht, wird die Pulsfolge verstärkt und als Heizstrom durch das Heizelement geleitet.
Die dargestellte Pulsfolge ist beispielhaft zu verstehen. Prinzipiell können z. B. auch komplexere Pulsfolgen gemäß dem oben angegebenen Prinzip programmiert und generiert werden. Ebenso ist es möglich, durch Rückkopplung über einen Messfühler zur Erfassung einer Temperatur oder einer Strömungsgeschwindigkeit eine Regelung der Flussrate zu erreichen. Hierbei kann eine Regelung der Flussrate z. B. dadurch erfolgen, dass Einfluss auf die Länge von Pulsen oder Pausen oder auf die Leistungsabgabe genommen wird: Eine derartige Regelung der Flussrate kann dann unter anderem auch mittels einer a-periodischen Pulsfolge bewirkt werden.

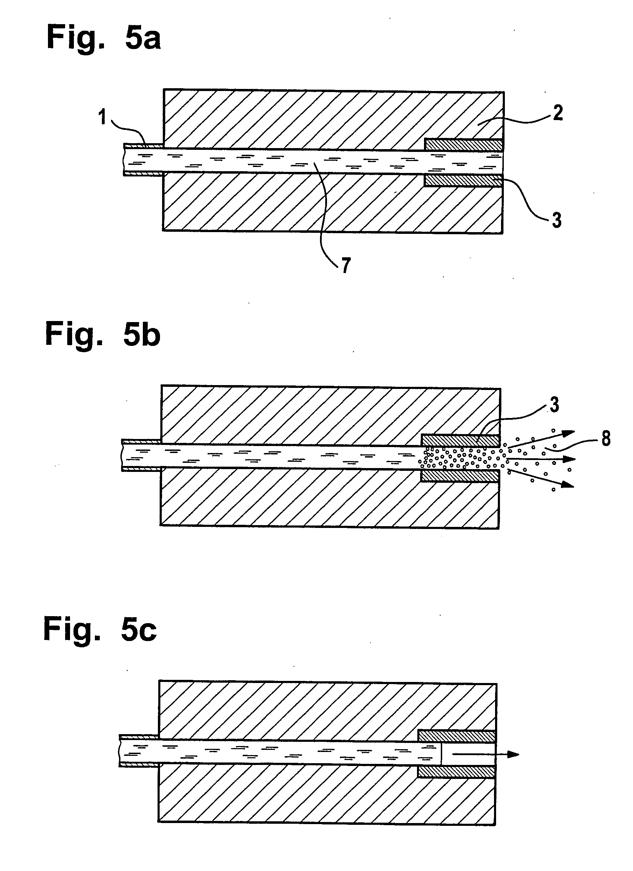

Figur 5 verdeutlicht noch mal schematisch den Ablauf eines Pumpenzyklusses. Zu Beginn des Pumpenzyklusses ist das fluidische Transportelement (1) innerhalb des Pumpenkörpers (2) im Wesentlichen vollständig mit einer Flüssigkeit (7) gefüllt. Im Bereich des Transportelementendes ist eine Widerstandsheizung angeordnet. Wird durch einen Puls die Heizung aktiviert, befindet sich die Heizung für einen Zeitraum T2 in einer Heizphase. Während der Heizphase T2 verdampft die Flüssigkeit aus dem Bereich des Transportelementendes. Ist der Dampf (8) aus dem Transportelement entwichen und die Heizphase T2 ist beendet, fließt Flüssigkeit in den Bereich des Transportelementendes nach. Die Pumpe befindet sich nun in einer Ruhephase T1. Während der Ruhephase T1 wird das Transportelementende erneut mit Flüssigkeit gefüllt, sodass sich ein Flüssigkeitsstroms innerhalb der Pumpe ausbildet. Aufgrund der nachfließenden Flüssigkeit entsteht eine Pumpwirkung, die ein Flüssigkeitstransport innerhalb des Systems bewirkt. Durch einen erneuten Heizpuls wiederholt sich der Pumpenzyklus entsprechend der Abbildung 5a bis 5c.

**Patentansprüche**

1. Mikropumpe mit einem Heizelement beinhaltend

- ein fluidisches Transportelement, das an ein Flüssigkeitsreservoir anschließbar ist und im angeschlossenen Zustand das Flüssigkeitsreservoir mit einem Verdampfungsbereich fluidisch verbindet,
- ein Heizelement, das sich im Bereich eines Transportelementendes befindet,
- eine Steuereinheit zur Steuerung des Heizelementes,
- wobei die Steuereinheit das Heizelement in der Weise durch Signale steuert, dass das Heizelement sich abwechselnd für einen Zeitraum T1 in einer Ruhephase oder für einen Zeitraum T2 in einer Heizphase befindet und sich die Ruhephase und die Heizphase im Wesentlichen periodisch abwechseln, so dass ein pulsierter Betrieb des Heizelementes erfolgt.

2. Mikropumpe gemäß Anspruch 1 bei dem die Flussrate der Mikropumpe durch die Länge der Zeiträume T1, T2 und durch die Heizleistung (HL) bestimmt wird und mindestens einer der Parameter (T1, T2, HL) durch die Steuereinheit veränderbar ist.

3. Mikropumpe gemäß Anspruch 1 bei der sich das Signal während der Heizphase T2 aus mehreren Signalen zusammensetzt.

4. Mikropumpe gemäß Anspruch 1 bei der das Heizelement innerhalb des Transportelementes positioniert ist.

**5.** Mikropumpe gemäß Anspruch 1 bei der das Heizelement außerhalb des Transportelementes positioniert ist.

**6.** Mikropumpe gemäß Anspruch 1 bei der das Transportelement aus Einzelfäden, die Litzen bilden, gebildet wird.

**7.** Mikropumpe gemäß Anspruch 1 bei der die Signale der Steuereinheit elektrische Signale sind.

**8.** Mikropumpe gemäß Anspruch 1 bei der das Heizelement eine Widerstandsheizung ist.

**9.** Mikropumpe gemäß Anspruch 1 bei der mindestens zwei Transportelemente vorhanden sind.

**10.** Mikropumpe gemäß Anspruch 9 bei der das zweite Transportelement mit dem ersten Transportelement sowie mit einem zweiten Flüssigkeitsreservoir fluidisch verbunden ist.

**11.** Mikropumpe gemäß Anspruch 1 oder 9 bei der mindestens zwei Heizelemente vorhanden sind.

**12.** Mikropumpe gemäß Anspruch 1 bei der das Transportelement durch einen Kanal gebildet wird.

**13.** Mikropumpe gemäß Anspruch 1 bei der das Transportelement durch eine mikroporöse Struktur gebildet wird.

**14.** Mikropumpe gemäß Anspruch 1, bei der die Transportelemente und/oder die Heizelemente mit mikrotechnischen Verfahren hergestellt werden.

**15.** Mikropumpe gemäß einer der vorhergehenden Ansprüche,

- die an einen Flüssigkeitsreservoir, in dem sich eine zu transportierende Flüssigkeit befindet, angeschlossen ist und das fluidische Transportelement mit der zu transportierenden Flüssigkeit zumindest zum Teil gefüllt ist.

**16.** Verfahren zum Betreiben einer Mikropumpe beinhaltend

- Aussenden eines ersten Signals, das eine Heizphase initiiert an ein Heizelement, so dass es zu einer Wärmeentwicklung in einem Endbereich eines fluidischen Transportelements einer Mikropumpe kommt, und

- Fortsetzen der Heizphase für einen definierten ZeitraumT2, der durch das ausgesendete Signal bestimmt wird,

- Verdampfen mindestens eines Teiles einer Flüssigkeit, die sich im Endbereich des Transportelementes befindet, durch die erzeugte Wärmemenge, wobei die

- im Wesentlichen vollständig verdampften Flüssigkeit aus dem Transportelement entweicht,

- Aussetzen der Heizphase für einen Zeitraum T1, so dass der Bereich des Transportelementendes abkühlt, wobei

- Flüssigkeit ins Transportelementende nachfließt, und

- Aussenden eines zweiten Signals nach dem Zeitraum T1, so dass erneut eine Heizphase initiiert und Flüssigkeit verdampft wird,

- Wiederholen der oben genannten Schritte, sodass sich aufgrund des Nachfließens der Flüssigkeit ins Transportelementende eine bestimmte Flussrate einstellt.

**17.** Verfahren gemäß Anspruch 16 bei dem das Nachfließen von Flüssigkeit ins Transportelementende im wesentlichen durch Kapillarkräfte im Transportelement verursacht wird.

**18.** Verfahren gemäß Anspruch 16 bei dem während des Betreibens der Pumpe das Heizelement in weniger als 20 % der Betriebszeit aktiviert ist.

**19.** Verfahren gemäß Anspruch 16 bei dem die im Wesentlichen vollständig verdampfte Flüssigkeit aus dem Transportelement in die Umgebung der Mikropumpe entweicht.

**20.** Verfahren gemäß Anspruch 16 bei dem die im Wesentlichen vollständig verdampfte Flüssigkeit aus dem Transportelement in einen Sorptionsbereich mit einem Sorptionsmittel entweicht.

**21.** Verfahren gemäß Anspruch 16 bei dem der Zeitabstand zwischen den Heizphasen so groß ist, dass ein Abkühlen des Transportelementendes im wesentlichen auf die Umgebungstemperatur erfolgt.

**22.** Mikropumpe gemäß Anspruch 1 bei der ein Verfahren nach einer der Ansprüche 15 - 21 angewendet wird.

**23.** Verfahren gemäß Anspruch 16 bei dem eine Mikropumpe nach einer der Ansprüche 1 - 15 verwendet wird.

**24.** Mikrodialysesystem mit einer Pumpe nach einem der Ansprüche 1 - 15, sowie einem Mikrodialysekatheter, der eine Mikrodialysemembran besitzt, an der die Pumpe eine Flüssigkeit vorbeitransportiert.

**25.** Mikrodialysesystem nach Anspruch 24, mit einem stromabwärts der Mikrodialysemembran angeordneten Sensor zur Detektion eines oder mehrerer Analyten in der Flüssigkeit.

**26.** Ultrafiltrationseinrichtung mit einer Pumpe nach einer der Ansprüche 1 - 15, sowie einem Ultrafiltrationskatheter, welcher eine Ultrafiltrationsmembran besitzt durch die Körperflüssigkeit eingezogen werden kann.

**27.** Ultrafiltrationseinrichtung nach Anspruch 26, mit einem stromabwärts der Ultrafiltrationsmembran angeordneten Sensor zur Detektion eines oder mehrerer Analyten in der Körperflüssigkeit.

# Fig. 1

# Fig. 2

# Fig. 3a

Heizstrom I

30    30    30

31    31    31

33    33

Zeit t

# Fig. 3b

Heizstrom I

30    30    30

31    31    31

33    33

Zeit t

# Fig. 3c

Heizstrom I

30    30

31    34    31

32    32

33    33

Zeit t

⬛ Heizimpulse

# Fig. 4a

# Fig. 4b

# Fig. 5a

# Fig. 5b

# Fig. 5c